# EUROPEAN PATENT SPECIFICATION

(11) **EP 4 470 571 B1**
(45) Date of publication and mention of the grant of the patent: **03.09.2025**
(21) Application number: 24177476.9
(22) Date of filing: 22.05.2024
(51) Int. Cl.: B08B 3/14, B08B 9/32, B67C 7/00, A61L 2/18, B65B 55/10

(54) **AN APPARATUS AND A PROCESS FOR STERILIZING AND RINSING CONTAINERS**
VORRICHTUNG UND VERFAHREN ZUM STERILISIEREN UND SPÜLEN VON BEHÄLTERN
APPAREIL ET PROCÉDÉ DE STÉRILISATION ET DE RINÇAGE DE RÉCIPIENTS

(30) Priority: 30.05.2023 IT 202300010905
(43) Date of publication of application: 04.12.2024
(73) Proprietor: Gea Procomac S.p.A., 43038 Sala Baganza (PR) (IT)
(72) Inventor: COMANI, Andrea, 43038 SALA BAGANZA (PARMA) (IT); FORNARI, Angelo, 43052 COLORNO (PARMA) (IT); BRICOLI, Barbara, 43014 MEDESANO (PARMA) (IT)
(74) Representative: Dondi, Silvia

(56) References cited:
- JP-A- 2003 137 228
- JP-A- 2011 218 353
- US-A1- 2013 092 196

## Description

The present invention relates to an apparatus and a process for sterilizing and rinsing containers.

In an aseptic bottling line, there is the need to sterilize the containers formed before they are filled.

In this context, the attention is focused on chemical sterilization, in which the external and internal surfaces of the containers are treated with hydrogen peroxide, acetic acid or peracetic acid.

After the chemical treatment, the containers are rinsed with sterile water.

The main issue of chemical sterilization is connected with the disposal of chemical residues and the consumption of water for the rinsing step.

In accordance with a known solution, the rinsing water is purified by an active carbon filter, and it is then partially recycled in the process.

In another solution, the rinsing water is passed through a catalyst and then in an ion exchange resin.

From document US 2013/092196 A1 it is known a method for sterilizing and rinsing containers in aseptic applications.

From document JP 2003 137228 A it is known an apparatus for sterilizing and rinsing containers which comprises a collector for recoverying the rinsing water at the rinsing carousel.

In this context, the object of the present invention is to provide an apparatus and a process for sterilizing and rinsing containers, which overcome the problems of the prior art cited above.

In particular, the object of the present invention is to propose an apparatus and a process for sterilizing and rinsing containers, which reduce the water consumption for the rinsing step.

Another object of the present invention is to propose an apparatus and a process for sterilizing and rinsing containers, having a lower environmental impact with respect to the known solutions.

The stated technical task and specified aims are substantially achieved by an apparatus and a process for sterilizing and rinsing containers as set out in the appended set of claims.

Further characteristics and advantages of the present invention will more fully emerge from the non-limiting description of a preferred but not exclusive embodiment of an apparatus and a process for sterilizing and rinsing containers, as illustrated in the accompanying drawings in which:
- figure 1 schematically illustrates an apparatus for sterilizing and rinsing containers, according to the present invention;
- figure 2 is a plain view of an embodiment of an apparatus for sterilizing and rinsing containers, according to the present invention;
- figure 3 illustrates the simplified structure of a part of the apparatus of figure 2 (focusing on the rinsing unit), in plain view;

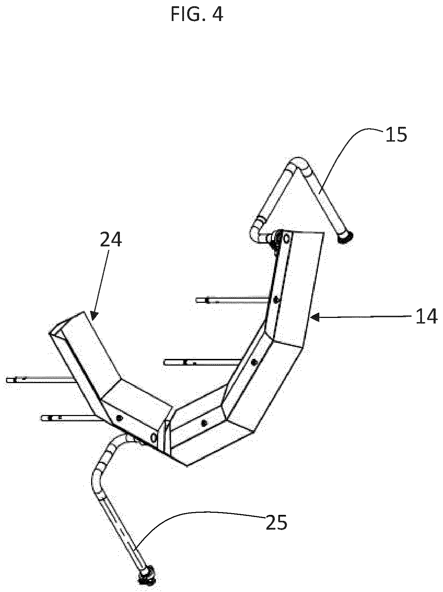

- figure 4 illustrates the collectors arranged at the rinsing unit of figure 3, in a perspective view.

With reference to the drawings, number 1 denotes an apparatus for sterilizing and rinsing containers 100, in particular made of a thermoplastic material.

The apparatus 1 comprises a sterilizing unit 10 and a rinsing unit 20 arranged downstream the sterilizing unit 10.

The sterilizing unit 10 is configured to treat the containers 100 by means of a sterilizing solution.

In particular, the apparatus 1 comprises a first tank 11 containing a sterilizing solution, which is a chemical compound based on one or more species of peroxides and/or organic acids.

In one example, the first tank 11 contains a peracetic acid solution.

The rinsing unit 20 is configured to rinse the containers 100 using sterile water.

In particular, the apparatus 1 comprises a **UHT** device 22 configured to provide sterile water, obtained by thermal treatment, to the rinsing unit 20. Preferably, the apparatus 1 comprises a second tank 21 arranged downstream the UHT device 22 so as to receive the sterile water from it.

In practice, the sterile water obtained in the UHT device 22 is supplied to the second tank 21.

In the rinsing unit 20 the containers 100 are made to advance along a path P.

The apparatus 1 further comprises dispensing means 23 in selective fluid communication with the UHT device 22.

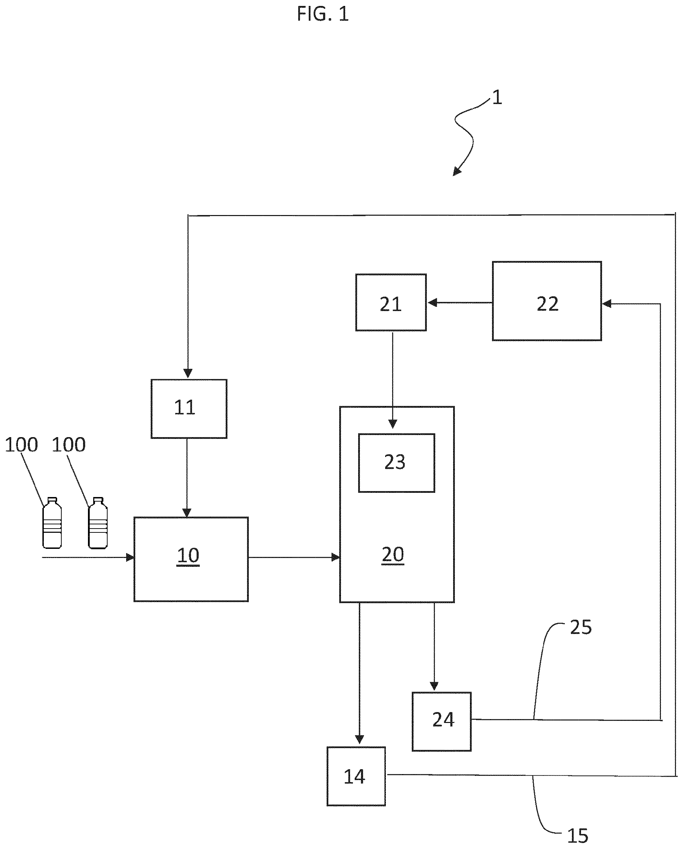

In one embodiment of the invention, illustrated in figure 1, the second tank 21 is configured to receive the sterile water from the UHT device 22 and to supply it to the dispensing means 23.

The dispensing means 23 are configured to dispense a first amount of sterile water towards the containers 100 in a first rinsing tract P1 of the path P and a second amount of sterile water towards the containers 100 in a second rinsing tract P2 of the path P.

In accordance with an embodiment of the invention, the dispensing means 23 are part of the rinsing unit 20.

Preferably, the dispensing means 23 comprise a plurality of nozzles.

More preferably, the nozzles are external to the containers 100 and are configured to dispense sterile water on internal walls of the containers 100. In an alternative embodiment, the nozzles are also configured to penetrate inside the containers 100 so as to dispense sterile water on internal walls of the containers 100.

When the containers 100 move in the rinsing unit 20 they advance along the first rinsing tract P1 and then along the second rinsing tract P2.

The second rinsing tract P2 is downstream the first rinsing tract P1 with respect to the advancement direction of the containers 100 along the path P in the rinsing unit 20.

In one example, the second rinsing tract P2 is immediately consecutive to the first rinsing tract P1. In another example, the second rinsing tract P2 is successive to the first rinsing tract P1 but separated from that.

Preferably, the dispensing means 23 are configured to dispense sterile water by a single dispensing pulse which supplies both the first amount of sterile water and the second amount of sterile water.

Alternatively, the dispensing means 23 are configured to dispense the first amount of sterile water with a first dispensing pulse, and the second amount of sterile water with a second dispensing pulse. In practice, the second dispensing pulse occurs after the first dispensing pulse has finished.

Advantageously, the apparatus 1 further comprises a recovery collector 24 arranged in the rinsing unit 20, in particular at the second rinsing tract P2 for collecting at least one part of the exhaust sterile water dropping from the containers 100 advancing along the second rinsing tract P2.

The recovery collector 24 is in selective fluid communication with the second tank 21 via the UHT device 22 so as to recover the collected exhaust sterile water dropping from the containers 100 in the second rinsing tract P2.

The apparatus 1 comprises a further recovery collector 14 arranged in the rinsing unit 20, in particular at the first rinsing tract P1 for collecting at least part of the exhaust sterile water dropping from the containers 100 advancing along the first rinsing tract P1.

The further recovery collector 14 is in selective fluid communication with the first tank 11 so as to recover the collected exhaust sterile water dropping from the containers 100 in the first rinsing tract P1.

In particular, the recovery collector 24 and the further recovery collector 14 are separated, i.e. they are not in fluid communication. As a matter of fact, they are configured to collect exhaust sterile water in two different zones of the rinsing unit 20, which correspond to different contents of peroxides.

Preferably, the apparatus 1 comprises a piping system 25 configured to establish a selective fluid communication between the recovery collector 24 and the UHT device 22, which in turn feeds the second tank 21. In particular, the piping system 25 is configured to channelize the exhaust sterile water recovered from the recovery collector 24 towards the UHT device 22.

For example, the piping system 25 comprises a pump.

Analogously, the apparatus 1 comprises a further piping system 15 configured to establish a selective fluid communication between the further recovery collector 14 and the first tank 11. In particular, the further piping system 15 is configured to channelize the exhaust sterile water recovered from the further recovery collector 14 towards the first tank 11.

For example, the further piping system 15 comprises another pump.

Preferably, the recovery collector 24 and the further recovery collector 14 are each designed as leak pans configured to collect dropping water.

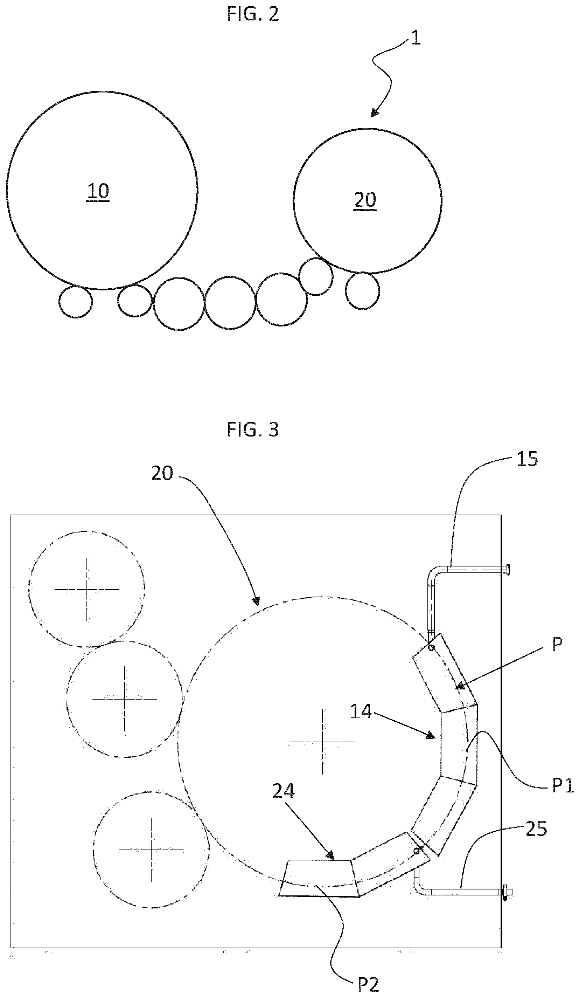

Said leak pans are respectively arranged below the first rinsing tract P1 and the second rinsing tract P2, as shown in figure 3.

According to one embodiment of the invention, illustrated in figure 2, the apparatus 1 is of the rotating type. Therefore, both the sterilizing unit 10 and the rinsing unit 20 comprise a rotating carousel.

In this embodiment, the path P along the rinsing unit 20 is a curvilinear path. The first rinsing tract P1 and the second rinsing tract P2 are arcuate portions of the curvilinear path P.

According to another embodiment of the invention (not illustrated), the apparatus 1 is of the linear type. Therefore, both the sterilizing unit 10 and the rinsing unit 20 comprise a linear conveyor or belt.

In this embodiment, the path P along the rinsing unit 20 is a linear path.

The first rinsing tract P1 and the second rinsing tract P2 are linear portions of the linear path P.

The process for sterilizing and rinsing containers, according to the present invention, is described hereafter.

The containers 100 are first sterilized with a sterilizing solution.

In the embodiment described and illustrated herewith, the containers 100 are sterilized in the sterilizing unit 10 by the sterilizing solution coming from the first tank 11.

After having been sterilized, the containers 100 are rinsed. According to one aspect of the invention, the containers 100 undergo two rinsing steps.

In particular, both the rinsing steps occur in the rinsing unit 20.

In a first rinsing tract P1 of the path P of the containers 100 in the rinsing unit 20, the containers 100 undergo a first rinsing step. For the first rinsing step it is employed a first amount of sterile water coming from the second tank 21.

The first amount of sterile water is dispensed towards the containers 100 when they are moving along the first rinsing tract P1.

In particular, the first amount of sterile water is dispensed by nozzles (not illustrated) which are in selective fluid communication with the UHT device 22 (and in particular with the second tank 21).

At least a part of exhaust sterile water used during the first rinsing step is recovered for being used for sterilizing new containers 100.

In particular, part of the exhaust sterile water dropping from the containers 100 which undergo the first rinsing step is collected by the further recovery collector 14 and sent back to the first tank 11.

This part of exhaust sterile water recovered from the first rinsing step is called here "first recovered water".

After the first rinsing step, the first recovered water was monitored and analyzed. The content of peroxides within the first recovered water is in the range of 200-1500 ppm.

It was noticed that the concentration of peroxides in the first recovered water was compatible for being mixed with the sterilizing solution of the first tank 11 without affecting it.

Thus, the first recovered water is used for sterilizing new containers 100 in the sterilizing unit 10.

After the first rinsing step, the process comprises a second rinsing step which is carried out in a second rinsing tract P2 of the path P.

For the second rinsing step it is employed a second amount of sterile water coming from the second tank 21.

The second amount of sterile water is dispensed towards the containers 100 when they are moving along the second rinsing tract P2.

In particular, the second amount of sterile water is dispensed by nozzles (not illustrated) which are in selective fluid communication with the **UHT** device 22 (and therefore with the second tank 21).

In one embodiment of the process, the dispensing means 23 (i.e. the nozzles) are configured to dispense the sterile water by a single dispensing pulse (without any interruption) that spans both the first rinsing step and the second rinsing step. In other words, there is no interruption between dispensing the first amount of sterile water and the second amount of sterile water.

In another embodiment of the process, the dispensing means 23 are configured to dispense the first amount of sterile water with a first dispensing pulse, and the second amount of sterile water with a second dispensing pulse. In practice, the second dispensing pulse occurs after the first dispensing pulse has finished.

At least a part of exhaust sterile water used during the second rinsing step is recovered for being used in rinsing new containers 100.

In particular, part of the exhaust sterile water dropping from the containers 100 which undergo the second rinsing step is collected by the recovery collector 24 and sent back to the UHT device 22.

This part of exhaust sterile water recovered from the second rinsing step is called here "second recovered water".

After the second rinsing step, the second recovered water was monitored and analyzed. The content of peroxides within the second recovered water is in the range of 10-100 ppm.

This concentration of peroxides is lower than the concentration of peroxides in the first recovered water. This is due to the fact that containers 100 undergoing the second rinsing have already been rinsed in the first rinsing step, thus they have lower peroxide residues.

In particular, it was noticed that the concentration of peroxides in the second recovered water was sufficiently low for being compatible with the UHT device 22 for generating sterile water.

Thus, the second recovered water is used for rinsing new containers 100 in the rinsing unit 20, in particular in the first rinsing tract P1 and/or in the second rinsing tract P2.

As said, only part of the exhaust sterile water used to rinse the containers 100 is recovered and may be used in the next rinsing cycles.

There is a part of exhaust sterile water which drops from the containers 100 and is neither collected by the recovery collector 24 nor by the further recovery collector 14. This part of exhaust sterile water may be discharged by means of a discharge outlet or may be channelized and reused in another way within the apparatus 1.

The characteristics of the apparatus and process for sterilizing and rinsing containers, according to the present invention, are clear, as are the advantages.

In particular, the invention allows a partial recovery of the exhaust sterile water used for rinsing the containers. Depending on the content of peroxides the recovered exhaust sterile water may be used for sterilizing or for rinsing new containers.

In particular, after the second rinsing step the content of peroxides is sufficiently lower for allowing the recovered exhaust sterile water to be used for next rinsing after having been thermally treated. There is no need to further treat or filter this recovered water since the content of peroxides is sufficiently low to be submitted as is to the UHT device.

The recovery of part of the sterile water for next rinsing steps is even more advantageous since the consumption of fresh water to produce sterile water is reduced.

Thus, the first recovered water is used for sterilizing new containers 100 in the sterilizing unit 10.

After the first rinsing step, the process comprises a second rinsing step which is carried out in a second rinsing tract P2 of the path P.

For the second rinsing step it is employed a second amount of sterile water coming from the second tank 21.

The second amount of sterile water is dispensed towards the containers 100 when they are moving along the second rinsing tract P2.

In particular, the second amount of sterile water is dispensed by nozzles (not illustrated) which are in selective fluid communication with the **UHT** device 22 (and therefore with the second tank 21).

In one embodiment of the process, the dispensing means 23 (i.e. the nozzles) are configured to dispense the sterile water by a single dispensing pulse (without any interruption) that spans both the first rinsing step and the second rinsing step. In other words, there is no interruption between dispensing the first amount of sterile water and the second amount of sterile water.

In another embodiment of the process, the dispensing means 23 are configured to dispense the first amount of sterile water with a first dispensing pulse, and the second amount of sterile water with a second dispensing pulse. In practice, the second dispensing pulse occurs after the first dispensing pulse has finished.

At least a part of exhaust sterile water used during the second rinsing step is recovered for being used in rinsing new containers 100.

In particular, part of the exhaust sterile water dropping from the containers 100 which undergo the second rinsing step is collected by the recovery collector 24 and sent back to the UHT device 22.

This part of exhaust sterile water recovered from the second rinsing step is called here "second recovered water".

After the second rinsing step, the second recovered water was monitored and analyzed. The content of peroxides within the second recovered water is in the range of 10-100 ppm.

This concentration of peroxides is lower than the concentration of peroxides in the first recovered water. This is due to the fact that containers 100 undergoing the second rinsing have already been rinsed in the first rinsing step, thus they have lower peroxide residues.

In particular, it was noticed that the concentration of peroxides in the second recovered water was sufficiently low for being compatible with the UHT device 22 for generating sterile water.

Thus, the second recovered water is used for rinsing new containers 100 in the rinsing unit 20, in particular in the first rinsing tract P1 and/or in the second rinsing tract P2.

As said, only part of the exhaust sterile water used to rinse the containers 100 is recovered and may be used in the next rinsing cycles.

There is a part of exhaust sterile water which drops from the containers 100 and is neither collected by the recovery collector 24 nor by the further recovery collector 14. This part of exhaust sterile water may be discharged by means of a discharge outlet or may be channelized and reused in another way within the apparatus 1.

The characteristics of the apparatus and process for sterilizing and rinsing containers, according to the present invention, are clear, as are the advantages.

In particular, the invention allows a partial recovery of the exhaust sterile water used for rinsing the containers. Depending on the content of peroxides the recovered exhaust sterile water may be used for sterilizing or for rinsing new containers.

In particular, after the second rinsing step the content of peroxides is sufficiently lower for allowing the recovered exhaust sterile water to be used for next rinsing after having been thermally treated. There is no need to further treat or filter this recovered water since the content of peroxides is sufficiently low to be submitted as is to the UHT device.

The recovery of part of the sterile water for next rinsing steps is even more advantageous since the consumption of fresh water to produce sterile water is reduced.

## Claims

1. An apparatus (1) for sterilizing and rinsing containers (100), comprising:
- a sterilizing unit (10) configured to treat the containers (100) by means of a sterilizing solution contained in a first tank (11), said sterilizing unit (10) comprising a corresponding rotating carousel;
- a rinsing unit (20) arranged downstream the sterilizing unit (10) and configured to rinse the containers (100) advancing along a path (P) with sterile water, said rinsing unit (20) comprising a corresponding rotating carousel, said path (P) along the rinsing unit (20) being a curvilinear path;
- a UHT device (22) configured to provide sterile water to the rinsing unit (20);
- dispensing means (23) in selective fluid communication with the UHT device (22) so as to dispense a first amount of sterile water towards the containers (100) in a first rinsing tract (P1) of the path (P) and a second amount of sterile water towards the containers (100) in a second rinsing tract (P2) of the path (P) that is downstream the first rinsing tract (P1), the first rinsing tract (P1) and the second rinsing tract (P2) being arcuate portions of the curvilinear path (P);
- a recovery collector (24) arranged at the second rinsing tract (P2) of the path (P) for collecting at least part of exhaust sterile water dropping from the containers (100) advancing along the second rinsing tract (P2), said recovery collector (24) being in selective fluid communication with the UHT device (22);
- a further recovery collector (14) arranged at the first rinsing tract (P1) of the path (P) for collecting at least part of the exhaust sterile water dropping from the containers (100) advancing along the first rinsing tract (P1), said further recovery collector (14) being in selective fluid communication with the first tank (11), said recovery collector (24) and the further recovery collector (14) being separated, that means not in fluid communication.

2. The apparatus (1) according to claim 1, comprising two distinct piping systems (25, 15) configured to establish a selective fluid communication respectively between the recovery collector (24) and the UHT device (22) and between the further recovery collector (14) and the first tank (11).

3. The apparatus (1) according to any one of claims 1 to 2, wherein the sterilizing unit (10) and the rinsing unit (20) comprise a corresponding linear conveyor or belt.

4. The apparatus (1) according to any of the preceding claims, further comprising a second tank (21) arranged downstream the UHT device (22) so as to receive the sterile water from it, said second tank (21) being configured to supply the sterile water to the dispensing means (23).

5. A process for sterilizing and rinsing containers (100) using an apparatus (1) according to any one of the preceding claims, the process comprising the following steps:
- sterilizing the containers (100) with a sterilizing solution coming from a first tank (11);
- after sterilization, subjecting the containers (100) to a first rinsing by dispensing towards them a first amount of sterile water from a UHT device (22) in a first rinsing tract (P1) of an advancement path (P) of the containers (100);
- after the first rinsing, subjecting the containers (100) to a second rinsing by dispensing towards them a second amount of sterile water from said UHT device (22) in a second rinsing tract (P2) of the path (P);
- collecting at least part of exhaust sterile water dropping from the containers (100) undergoing the first rinsing and sending it to the first tank (11);
- collecting at least part of exhaust sterile water dropping from the containers (100) undergoing the second rinsing and sending it to the UHT device (22).

6. The process of claim 5, wherein the exhaust sterile water which is sent to the UHT device (22) is used in rinsing new containers (100) advancing along the path (P).

7. The process of claim 5 or 6, wherein the first rinsing step and the second rinsing step are performed by dispensing means (23) which are configured to dispense sterile water by a single dispensing pulse which spans both the first rinsing step and the second rinsing step.

8. The process of claim 7, wherein the dispensing means (23) are configured to dispense the first amount of sterile water and the second amount of sterile water without any interruption.

9. The process of any one of the claims 5 to 8, wherein the sterile water obtained in the UHT device (22) is sent to a second tank (21) which in turn is configured to dispense the sterile water towards the containers (100) during the first rising and the second rinsing.

10. The process according to any one of claims 5 to 9, wherein the exhaust sterile water collected in the first rinsing tract (P1) is mixed with the sterilizing solution of the first tank (11) for being used in sterilizing new containers (100).

## Patentansprüche

1. Vorrichtung (1) zum Sterilisieren und Spülen von Behältern (100), umfassend:
- eine Sterilisationseinheit (10), die ausgelegt ist, um die Behälter (100) mittels einer Sterilisationslösung zu behandeln, die in einem ersten Tank (11) enthalten ist, wobei die Sterilisationseinheit (10) ein entsprechendes rotierendes Karussell umfasst;
- eine Spüleinheit (20), die stromabwärts der Sterilisationseinheit (10) angeordnet und ausgelegt ist, um die Behälter (100), die sich entlang eines Pfades (P) vorwärts bewegen, mit sterilem Wasser zu spülen, wobei die Spüleinheit (20) ein entsprechendes rotierendes Karussell umfasst, wobei der Pfad (P) entlang der Spüleinheit (20) ein gekrümmter Pfad ist;
- eine UHT-Einrichtung (22), die ausgelegt ist, um der Spüleinheit (20) steriles Wasser bereitzustellen;
- Abgabemittel (23) in selektiver Fluidkommunikation mit der UHT-Einrichtung (22), um eine erste Menge an sterilem Wasser in Richtung der Behälter (100) in einem ersten Spülweg (P1) des Pfads (P) und eine zweite Menge an sterilem Wasser in Richtung der Behälter (100) in einem zweiten Spülweg (P2) des Pfads (P) abzugeben, der stromabwärts des ersten Spülwegs (P1) liegt, wobei der erste Spülweg (P1) und der zweite Spülweg (P2) gekrümmte Abschnitte des gekrümmten Pfads (P) sind;
- einen Rückgewinnungssammler (24), der an dem zweiten Spülweg (P2) des Pfads (P) angeordnet ist, um zumindest einen Teil des sterilen Abwassers zu sammeln, das aus den Behältern (100) fällt, die sich entlang des zweiten Spülwegs (P2) vorwärts bewegen, wobei der Rückgewinnungssammler (24) in selektiver Fluidkommunikation mit der UHT-Einrichtung (22) steht;
- einen weiteren Rückgewinnungssammler (14), der an dem ersten Spülweg (P1) des Pfads (P) angeordnet ist, um zumindest einen Teil des sterilen Abwassers zu sammeln, das aus den Behältern (100) fällt, die sich entlang des ersten Spülwegs (P1) vorwärts bewegen, wobei der weitere Rückgewinnungssammler (14) in selektiver Fluidkommunikation mit dem ersten Tank (11) steht, wobei der Rückgewinnungssammler (24) und der weitere Rückgewinnungssammler (14) getrennt sind, d. h. nicht in Fluidkommunikation stehen.

2. Vorrichtung (1) nach Anspruch 1, umfassend zwei getrennte Rohrleitungssysteme (25, 15), die ausgelegt sind, um jeweils eine selektive Fluidkommunikation zwischen dem Rückgewinnungssammler (24) und der UHT-Einrichtung (22) und zwischen dem weiteren Rückgewinnungssammler (14) und dem ersten Tank (11) herzustellen.

3. Vorrichtung (1) nach einem der Ansprüche 1 bis 2, wobei die Sterilisationseinheit (10) und die Spüleinheit (20) einen entsprechenden linearen Förderer oder Riemen umfassen.

4. Vorrichtung (1) nach einem der vorhergehenden Ansprüche, ferner umfassend einen zweiten Tank (21), der stromabwärts der UHT-Einrichtung (22) angeordnet ist, um das sterile Wasser von dieser aufzunehmen, wobei der zweite Tank (21) ausgelegt ist, um den Abgabemitteln (23) das sterile Wasser zuzuführen.

5. Verfahren zum Sterilisieren und Spülen von Behältern (100) unter Verwendung einer Vorrichtung (1) nach einem der vorhergehenden Ansprüche, wobei das Verfahren die folgenden Schritte umfasst:
- Sterilisieren der Behälter (100) mit einer Sterilisationslösung, die aus einem ersten Tank (11) kommt;
- nach der Sterilisation, Unterziehen der Behälter (100) einer ersten Spülung durch Abgeben zu ihnen hinführend von einer ersten Menge an sterilem Wasser von einer UHT-Einrichtung (22) in einem ersten Spülweg (P1) eines Vorschubpfads (P) der Behälter (100);
- nach der ersten Spülung, Unterziehen der Behälter (100) einer zweiten Spülung durch Abgeben zu ihnen hinführend von einer zweiten Menge an sterilem Wasser von der UHT-Einrichtung (22) in einem zweiten Spülweg (P2) des Pfades (P);
- Sammeln mindestens eines Teils des sterilen Abwassers, das aus den Behältern (100) fällt, die der ersten Spülung unterzogen werden, und Senden desselben an den ersten Tank (11);
- Sammeln mindestens eines Teils des sterilen Abwassers, das aus den Behältern (100) fällt, die der zweiten Spülung unterzogen werden, und Senden desselben an die UHT-Einrichtung (22).

6. Verfahren nach Anspruch 5, wobei das sterile Abwasser, das an die UHT-Einrichtung (22) gesendet wird, zum Spülen neuer Behälter (100) verwendet wird, die sich entlang des Pfades (P) vorwärts bewegen.

7. Verfahren nach Anspruch 5 oder 6, wobei der erste Spülschritt und der zweite Spülschritt durch Abgabemittel (23) durchgeführt werden, die dazu ausgelegt sind, steriles Wasser durch einen einzigen Abgabeimpuls abzugeben, der sowohl den ersten Spülschritt als auch den zweiten Spülschritt überspannt.

8. Verfahren nach Anspruch 7, wobei die Abgabemittel (23) dazu ausgelegt sind, die erste Menge an sterilem Wasser und die zweite Menge an sterilem Wasser ohne Unterbrechung abzugeben.

9. Verfahren nach einem der Ansprüche 5 bis 8, wobei das in der UHT-Einrichtung (22) erhaltene sterile Wasser in einen zweiten Tank (21) gesendet wird, der wiederum dazu ausgelegt ist, das sterile Wasser während der ersten Spülung und der zweiten Spülung in Richtung der Behälter (100) abzugeben.

10. Verfahren nach einem der Ansprüche 5 bis 9, wobei das in dem ersten Spülweg (P1) gesammelte sterile Abwasser mit der Sterilisationslösung des ersten Tanks (11) gemischt wird, um beim Sterilisieren neuer Behälter (100) verwendet zu werden.

## Revendications

1. Appareil (1) de stérilisation et de rinçage de récipients (100), comprenant :
- une unité de stérilisation (10) configurée pour traiter les récipients (100) au moyen d'une solution stérilisante contenue dans un premier réservoir (11), ladite unité de stérilisation (10) comprenant un carrousel rotatif correspondant ;
- une unité de rinçage (20) disposée en aval de l'unité de stérilisation (10) et configurée pour rincer les récipients (100) avançant le long d'un parcours (P) avec de l'eau stérile, ladite unité de rinçage (20) comprenant un carrousel rotatif correspondant, ledit parcours (P) le long de l'unité de rinçage (20) étant un parcours curviligne ;
- un dispositif UHT (22) configuré pour fournir de l'eau stérile à l'unité de rinçage (20) ;
- des moyens de distribution (23) en communication fluidique sélective avec le dispositif UHT (22) de manière à distribuer une première quantité d'eau stérile vers les récipients (100) dans une première voie de rinçage (P1) du parcours (P) et une seconde quantité d'eau stérile vers les récipients (100) dans une seconde voie de rinçage (P2) du parcours (P) étant en aval de la première voie de rinçage (P1), la première voie de rinçage (P1) et la seconde voie de rinçage (P2) étant des parties arquées du parcours curviligne (P) ;
- un collecteur de récupération (24) disposé en correspondance de la seconde voie de rinçage (P2) du parcours (P) pour recueillir au moins une partie de l'eau stérile d'échappement tombant des récipients (100) avançant le long de la seconde voie de rinçage (P2), ledit collecteur de récupération (24) étant en communication fluidique sélective avec le dispositif UHT (22) ;
- un collecteur de récupération (14) supplémentaire disposé en correspondance de la première voie de rinçage (P1) du parcours (P) pour recueillir au moins une partie de l'eau stérile d'échappement tombant des récipients (100) avançant le long de la première voie de rinçage (P1), ledit collecteur de récupération (14) supplémentaire étant en communication fluidique sélective avec le premier réservoir (11), ledit collecteur de récupération (24) et le collecteur de récupération (14) supplémentaire étant séparés, c'est-à-dire n'étant pas en communication fluidique.

2. Appareil (1) selon la revendication 1, comprenant deux systèmes de tuyauterie distincts (25, 15) configurés pour établir une communication fluidique sélective respectivement entre le collecteur de récupération (24) et le dispositif UHT (22) et entre le collecteur de récupération (14) supplémentaire et le premier réservoir (11).

3. Appareil (1) selon l'une quelconque des revendications 1 à 2, dans lequel l'unité de stérilisation (10) et l'unité de rinçage (20) comprennent un transporteur ou une courroie linéaire correspondant(e).

4. Appareil (1) selon l'une quelconque des revendications précédentes, comprenant en outre un second réservoir (21) disposé en aval du dispositif UHT (22) de manière à en recevoir l'eau stérile, ledit second réservoir (21) étant configuré pour fournir l'eau stérile aux moyens de distribution (23).

5. Procédé de stérilisation et de rinçage de récipients (100) à l'aide d'un appareil (1) selon l'une quelconque des revendications précédentes, le procédé comprenant les étapes suivantes :
- stériliser les récipients (100) avec une solution stérilisante provenant d'un premier réservoir (11) ;
- après la stérilisation, soumettre les récipients (100) à un premier rinçage en distribuant vers ceux-ci une première quantité d'eau stérile provenant d'un dispositif UHT (22) dans une première voie de rinçage (P1) d'un parcours d'avancement (P) des récipients (100) ;
- après le premier rinçage, soumettre les récipients (100) à un second rinçage en distribuant vers ceux-ci une seconde quantité d'eau stérile provenant dudit dispositif UHT (22) dans une seconde voie de rinçage (P2) du parcours (P) ;
- recueillir au moins une partie de l'eau stérile d'échappement tombant des récipients (100) soumis au premier rinçage et l'envoyer dans le premier réservoir (11) ;
- recueillir au moins une partie de l'eau stérile d'échappement tombant des récipients (100) soumis au second rinçage et l'envoyer au dispositif UHT (22).

6. Procédé selon la revendication 5, dans lequel l'eau stérile d'échappement étant envoyée au dispositif UHT (22) est utilisée pour le rinçage de nouveaux récipients (100) avançant le long du parcours (P).

7. Procédé selon la revendication 5 ou 6, dans lequel la première étape de rinçage et la seconde étape de rinçage sont effectuées par des moyens de distribution (23) qui sont configurés pour distribuer de l'eau stérile par une seule impulsion de distribution qui englobe à la fois la première étape de rinçage et la seconde étape de rinçage.

8. Procédé selon la revendication 7, dans lequel les moyens de distribution (23) sont configurés pour distribuer la première quantité d'eau stérile et la seconde quantité d'eau stérile sans aucune interruption.

9. Procédé selon l'une quelconque des revendications 5 à 8, dans lequel l'eau stérile obtenue dans le dispositif UHT (22) est envoyée dans un second réservoir (21) qui est à son tour configuré pour distribuer l'eau stérile vers les récipients (100) pendant le premier rinçage et le second rinçage.

10. Procédé selon l'une quelconque des revendications 5 à 9, dans lequel l'eau stérile d'échappement recueillie dans la première voie de rinçage (P1) est mélangée à la solution de stérilisation du premier réservoir (11) pour être utilisée dans la stérilisation de nouveaux récipients (100).
